# EUROPEAN PATENT SPECIFICATION

(11) **EP 2 062 571 B1**
(45) Date of publication and mention of the grant of the patent: **14.11.2012**
(21) Application number: 08020342.5
(22) Date of filing: 21.11.2008
(51) Int. Cl.: A61K 9/107, A61K 9/48, A61K 47/10, A61K 47/26, A61K 47/44

(54) **Self-emulsifying pharmaceutical composition with enhanced bioavailability**
Selbstemulgierende pharmazeutische Zusammensetzung mit verbesserter Bioverfügbarkeit
Composition pharmaceutique à auto-émulsion avec une biodisponibilité améliorée

(30) Priority: 21.11.2007 US 944239
(43) Date of publication of application: 27.05.2009
(73) Proprietor: INNOPHARMAX INC., Taipei 114 (TW)
(72) Inventor: Hao, Wei-Hua, Taipei 114, Taiwan, R.O.C. (TW); Wang, Jong-Jing, Taipei 114, Taiwan, R.O.C. (TW); Hsu, Chang-Shan, Taipei 114, Taiwan, Taiwan R.O.C. (TW)
(74) Representative: Becker Kurig Straus

(56) References cited:
- EP-A- 0 274 870
- EP-A- 0 985 412
- WO-A-98/33512
- WO-A-98/40094
- FR-A- 2 775 596

## Description

The invention pertains to an oral self-emulsifying pharmaceutical composition containing a lipophilic drug, a hydrophilic carrier, and a surfactant. Said composition immediately self-emulsifies to micromicelles at a size of about 10 to about 800 nm once it contacts the gastrointestinal fluid.

### BACKGROUND OF THE INVENTION

Oral drug administration system is the long-term and most generally accepted administration route for treating diseases. However, about 50% of the drugs in all encounter limitation in oral administration due to high liposolubility. Moreover, about 40% of the newly developed drugs are liposoluble. Since the granules of most lipophilic drugs are hardly infiltrated by gastrointestinal fluids, they exhibit poorer solubility and release rate when administered as conventional tablets or capsules, and thus exhibit lower bioavailability. Furthermore, drug absorption in different individuals might differ significantly due to differences in gastrointestinal function and food intake. Therefore, it is rather difficult to determine and control the dosage. Given the above, improving the absorption of orally administered drugs is the key point in solving the problem of the low bioavailability of poorly soluble drugs.

To date, the following methods have been used to improve the bioavailability of poorly soluble drugs:
a) Converting poorly soluble drugs into soluble salts or esters.
b) Reducing the particle size and increasing the total surface area to enhance a drug's dissolution. It is normally done by mechanical processes.
c) Increasing the solubility of drug in water by adding solubilizing agents.
d) "Solid dispersion" (SDS) technique, which produce a homogeneously dispersed solid dispersion system by mixing one or more active ingredients and excipients. Commonly used methods are solvent method, melting method, solvent-spray method, and grinding method. The advantage of this technique is that it improves the dissolution and bioavailability of drugs.

However, drawbacks have been found with the above methods. For example, though poorly soluble weak acid or base drugs can be converted to soluble salts for drug administration, said soluble salts may turn back to poorly soluble weak acid or base drugs due to the pH change in gastrointestinal tract, thus resulting in precipitation of drugs. For another instance, the amount of carriers used in solid dispersions technique is often large, and thus if the dosage of major ingredient is high, the tablets or capsules formed will be large in volume and difficult to swallow. Moreover, since the carriers used are usually expensive and freeze-drying or spray-drying method requires particular facilities and processes, the production cost is rather high. Though traditional solvent method can be adopted instead, it is difficult to deal with co-precipitates with high viscosity. Recently, melting method has been utilized to directly fill the capsules. However, the water in the shell of the capsules may influence the stability of the solid dispersions. Moreover, since poorly soluble drugs may not exhibit high permeability in gastrointestinal tract, it is important to avoid crystallization of the drugs as a result of their incapability of being immediately absorbed after forming supersaturated solution.

Recently, there have been great interests in preparing oral formulations using lipid-based formulations, and a new technique called "self-emulsifying/microemulsifying drug delivery system (SEDDS/SMEDDS)" has been developed and used to improve the water solubility and bioavailability of hydrophobic drugs after oral administration. Normally the SEDDS/SMEDDS is composed of oil, a surfactant, a cosurfactant or solubilizer, and a drug. The underlying principle of said system is that when the SEDDS/SMEDDS contacts water, it spontaneously forms oil-in-water microemulsions under mild mechanical agitation. Consequently, a drug can be formulated so as to dissolve in a liquid-based formulation that does not contain an aqueous phase. It can then be filled into soft/hard capsules to form solid oral formulations. After being oral administered and contacting gastrointestinal fluids, said formulation is capable of self-emulsifying into microemulsions immediately so as to facilitate the dispersion, dissolution, stability and absorption of the drug, thus improving the bioavailability of said drug. Compared with emulsions/microemulsions, the SEDDS/SMEDDS not only has the same advantage of facilitating the solubility of hydrophobic drugs, but also overcomes the drawback of the layering of emulsions after sitting for a long time. The SEDDS/SMEDDS can be easily stored since it belongs to a thermodynamics-stable system. Furthermore, since the process for its production is easy and convenient, the SEDDS/SMEDDS is becoming an important field in pharmaceutical development.

Emulsions/microemulsions formed by the SEDDS/SMEDDS exhibit good thermodynamics stability and optical transparency. The major difference between the above microemulsions and common emulsions lies in the particle size of droplets. The size of the droplets of common emulsion ranges between 0.2 and 10 µm, and that of the droplets of microemulsion formed by the SMEDDS generally ranges between 2 and 100 nm (such droplets are called droplets of nano particles). Since the particle size is small, the total surface area thereof for absorption and dispersion is significantly larger than that of solid dosage form and it can easily penetrate the gastrointestinal tract and be absorbed. The bioavailability of the drug is therefore improved. In addition to enhancing the bioavailability of hydrophobic drugs, SEDDS/SMEDDS is also capable of embedding peptide/protein drugs in the oil phase of droplets so that the drugs will not be decomposed by enzymes in the gastrointestinal tract.

Despite the above advantages, the SEDDS/SMEDDS still has many drawbacks. SEDDS/SMEDDS is composed of an oil, a surfactant, a solubilizer, and a drug. The solubilizer, which is generally an organic solvent, volatilizes and decreases extremely easily in the form of either a solution or a capsule, and that results in the destruction of the balance between the phases, the precipitation of drugs, or the change in the size of the droplets, and affects the bioavailability of the drug. In addition, since the drugs are required to be soluble in oil phase system, only Class IV drugs, which have an extremely low water solubility, are more suited to be used in the SEDDS/SMEDDS. Furthermore, the commonly used oil, such as castor oil, is highly toxic. Since the oil excipient contained in the SEDDS/SMEDDS is easily denatured due to oxidation, and it is difficult to control the particle sizes of the emulsions formed by the utilization of the oil excipient, and the packaging and storage of the SEDDS/SMEDDS required strict control of humidity and temperature, thus pushing up the production cost. Therefore, there is still a need to develop a new dosage form to improve the solubility and bioavailability of hydrophobic drugs.

The present invention provides a pharmaceutical composition that improves the solubility and bioavailability of lipophilic drugs. The system used in the present invention well exhibit the function of SEDDS/SMEDDS in the absence of a traditional oil phase. Furthermore, the composition can be easily prepared and is convenient to take. It also exhibits good stability during long-term storage.

### SUMMARY OF THE INVENTION

One object of the invention is to provide an orally administered pharmaceutical composition to enhance the bioavailability of a drug, which comprises a therapeutically effective amount of a lipophilic drug, a hydrophilic carrier, and a surfactant, wherein the HLB value of said composition ranges from about 8 to about 15.

Another object of the invention is to provide an emulsion/microemulsion which is formed by the oral pharmaceutical composition of the invention.

Another object of the invention is to provide a method for preparing the oral pharmaceutical composition of the invention, comprising dissolving a drug in a hydrophilic solvent carrier, adding more than one surfactant, and adjusting the HLB value so that it ranges from about 8 to about 15.

A further object of the invention is to provide a method for enhancing the bioavailability of a lipophilic drug in a patient undergoing therapy, comprising orally administering to said patient the pharmaceutical composition of the present invention.

Still another object of the invention is to provide a method of administering a pharmaceutical active ingredient to a host to increase the bioavailability of the pharmaceutical active ingredient, which comprises the steps of: a) providing an oral pharmaceutical composition of the invention for oral administration; and b) administering said composition to said host for ingestion, whereby said composition contacts the biological fluids of the body and increases the bioavailability of the pharmaceutical active ingredient.

### BRIEF DESCRIPTION OF THE DRAWINGS

Figure 1 shows the *in vitro* dissolution curves of the Formulations (II)-1 to -3.

Figure 2 shows the *in vitro* dissolution curves of the Formulations (III)-1 to -3.

Figure 3 shows the *in vitro* dissolution curves of the Formulations (IV)-1 to -3.

Figure 4 shows the *in vitro* dissolution curves of the Formulations (V)-1 to -3.

Figure 5 shows the *in vitro* dissolution curves of the Tacrolimus Formulations I and II.

Figure 6 shows the blood concentration versus time curves of Formulation (V)-3 soft capsule, Formulation (I)-1 soft capsule, and Formulation (I)-1 hard capsule.

### DETAILED DESCRIPTION OF THE INVENTION

Unless otherwise defined herein, scientific and technical terms used in connection with the present invention shall have the meanings that are commonly understood by those of ordinary skill in the art. The meaning and scope of the terms should be clear; however, in the event of any latent ambiguity, definitions provided herein take precedence over any dictionary or extrinsic definition.

Unless otherwise required by context, singular terms shall include the plural and plural terms shall include the singular.

The present invention provides an orally administered pharmaceutical composition that improves the solubility and bioavailability of lipophilic drugs, which comprises a pharmaceutically effective amount of a lipophilic drug, a hydrophilic solvent carrier, and a surfactant, wherein the HLB value of said composition ranges from about 8 to about 15. When the pharmaceutical composition of the present invention comes into contact with the gastrointestinal fluid, it spontaneously emulsifies forming micromicelles with the drug contained therein in the absence of the easily oxidized oil phase used in the conventional oil-rich part of the ternary system to form emulsions/microemulsions. For example, the oil phase may be olive oil, corn oil, soybean oil, canola oil, sunflower oil, or medium chain triglyceride oil.

The term "therapeutically effective amount" should be understood as meaning a dose of the drug effective in exerting a therapeutic effect. For an oral preparation of the invention, the term "therapeutically effective amount" means a dose of the drug which, after absorption into the body through the walls of GI tract, yields a drug concentration in the blood effective in exerting a therapeutic effect on a target organ. Persons of ordinary skill in the art will understand that the amounts of the drug presented in the composition vary with the particular situation, including but not limited to, the mode of administration, the size, age and condition of the subject and the like. Moreover, these effective amounts can be easily determined by the physician without undue experimentation. It is preferred that the drug is present in amounts ranging from about 0.1% to about 50% by weight of the composition and more preferably in an amount ranging from about 1% to about 40% by weight.

According to the present invention, the lipophilic drugs include, but are not limited to, immune drugs, anti-infection drugs, anti-hypertensive drugs, blood lipid-lowering drugs, antacids, anti-inflammatory substances, coronary vasodilators, cerebral vasodilators, psychotropics, anti-neoplastics, stimulants, anti-histamines, laxatives, decongestants, vitamins, gastrointestinals, anti-diarrheal preparations, anti-anginal drugs, vasodilators, anti-arrythmics, anti-migraine drugs, anti-coagulants and anti-thrombotic drugs, analgesics, anti-pyretics, hypnotics, sedatives, anti-emetics, anti-nauseants, anti-convulsants, anti-epileptics, neuromuscular drugs, drugs acting on the CNS (Central Nervous System), hyper-and hypoglycemic agents, thyroid and anti-thyroid preparations, diuretics, anti-spasmodics, uterine relaxants, mineral and nutritional additives, anti-obesity drugs, anabolic drugs, anti-asmatics, expectorants, cough drugs or substants acting locally in the mouth, or combination thereof and the like. The composition of the invention may contain a combination of more than one active ingredient. The preferred embodiments of the drug are cyclosporine, tacrolimus, ibuprofen, ketoprofen, nifedipine, amlodipine, and simvastatin.

The hydrophilic carrier used in the present invention must be non-toxic and well tolerated physiologically. In addition, the carrier should allow the incorporation of the drug into the carrier. According to the present invention, the hydrophilic carrier includes, but is not limited to, ethanol, isopropanol, propylene glycol, and polyethylene glycol (such as PEG200, PEG300, PEG400, PEG600, PEG 1000, PEG2000, PEG3000, PEG4000, PEG6000, or PEG8000). Any of the above-mentioned carriers can be used alone or in combination with one or more carriers. In the composition of the present invention, it is preferred that the carrier is present in amounts ranging from about 1% to about 30% by weight of the composition and more preferably in an amount ranging from about 2% to about 20% by weight.

The surfactant used in the present invention may be any of those known in the art, which includes, but is not limited to, cationic surfactants, anionic surfactants, and nonionic surfactants. The surfactant used in the present invention should possess an HLB (Hydrophilic Lipophilic Balance) value of greater than about 2 according to the HLB system which is well known to those skilled in the art. The HLB value provides a means for ranking surfactants according to the balance between the hydrophilic and lipophilic portions of the surfactant agent. That is, the higher the HLB value, the more hydrophilic the surfactant agent. Typically, the surfactant used in the present invention has a HLB value ranging from about 2 to about 18. The preferred embodiments of the surfactant are PEG 40 hydrogenated castor oil, polysorbate, cocamidopropyl betaine, glyceryl cocoate, PEG 6 caprylic/capric glycerides, Poloxmer, Labrafil M1944CS, Labrafil M2125CS, Labrasol, Cremophor EL, Cremophor RH, Brij, and Spans. Any of the above-mentioned surfactants can be used alone or in combination with one or more surfactants. In the composition of the present invention, it is preferred that the surfactant is present in amounts ranging from about 10% to about 90% by weight of the composition and more preferably in an amount ranging from about 20% to about 85% by weight.

An essential aspect of the pharmaceutical composition of the present invention is that it forms an emulsion/microemulsion when placed in contact with an aqueous solution, e.g., gastrointestinal fluid. The microemulsion thus formed is thermodynamically stable when it comes into contact with the gastrointestinal fluids of mammals. However, until the composition comes into contact with the gastrointestinal fluid, it is not an emulsion/microemulsion. If an emulsion/microemulsion is formed, it consists of substantially uniform and spherical droplets. The particle size of the droplets in the present microemulsion is less than about 800 nm, preferably about 10 nm to about 800 nm.

The pharmaceutical composition of the present invention can be prepared by mixing the lipophilic drug, the hydrophilic carrier and the surfactant by agitation. Preferably, one or more surfactants are mixed, and one or more hydrophilic carriers are further added to the above mixture in a beaker or flask at room temperature by a magnetic stir or agitator to obtain a homogeneous solution. One or more drugs are then added and followed by further agitation until a clear solution is obtained so as to form a mixture having a HLB value that ranges between about 8 and about 15. Note that incorporation of air into the solution while mixing should be avoided.

For oral administration of the composition having the lipophilic drug, the composition is preferably encapsulated in a sealed soft or hard capsule. The capsule is typically of a kind which is dissolved in a particular region of the GI tract releasing its content there. An example of such a capsule is an enterice-coated soft or hard gelatin capsule. Enteric coating, as known per se, is coating with a substance or a combination of substances that resists dissolution in gastric fluid but disintegrates in the intestine.

The present invention is explained in greater detail in the following non-limiting analytical methods and results obtained therefrom:

### Example 1

| Ingredients | mg/capsule | |
|---|---|---|
| | F(I)-1 | F(I)-2 |
| Cyclosporine | 100 | 100 |
| EtOH | 117.6 | 141 |
| Tween80 | 758 | 758 |
| TOTAL | 975.6 | 999 |

Cyclosporine was added into and mixed with Tween80 and ethanol, and the mixtures were agitated until clear solutions were obtained. The formulations F(I)-1 and F(I)-2 obtained have the HLB values of 14.0 and 13.9, respectively. 975.6 mg and 999 mg respectively of the clear solutions of the two formulations were filled into capsules for further tests.

### Example 2

| Ingredients | mg/capsule |
|---|---|
| | F(I)-3 |
| Cyclosporine | 100 |
| EtOH | 117 |
| Labrasol | 758 |
| TOTAL | 975 |

Cyclosporine was added into and mixed with labrasol and ethanol, and the mixture was agitated until a clear solution was obtained. The formulation F(I)-3 obtained has the HLB values of 13.1. 975 mg of the clear solution of the formulation were filled into capsules for further tests.

### Example 3

| Ingredients | mg/capsule | | |
|---|---|---|---|
| | F(II)-1 | F(II)-2 | F(II)-3 |
| Cyclosporine | 100.0 | 100.0 | 100.0 |
| EtOH | 117.5 | 117.5 | 117.5 |
| Labrafil M2125CS | 84.5 | 189.5 | 324.5 |
| Tween80 | 758.0 | 758.0 | 758.0 |
| TOTAL | 1060 | 1165 | 1300 |

Cyclosporine was added into and mixed with Labrafil M2125CS, Tween80 and ethanol, and the mixtures were agitated until clear solutions were obtained. The formulations F(II)-1, F(II)-2 and F(II)-3 obtained have the HLB values of 13.1, 12.2, and 11.3, respectively. 1060 mg, 1165 mg, and 1300 mg respectively of the clear solutions of the three formulations were filled into capsules for further tests.

### Example 4

| Ingredients | mg/capsule | | |
|---|---|---|---|
| | F(III)-1 | F(III)-2 | F(III)-3 |
| Cyclosporine | 100.0 | 100.0 | 100.0 |
| EtOH | 117.5 | 117.5 | 117.5 |
| Propylene glycol | 189.5 | 324.5 | 454.5 |
| Tween80 | 758.0 | 758.0 | 758.0 |
| TOTAL | 1165 | 1300 | 1430 |

Cyclosporine was added into and mixed with propylene glycol, Tween80 and ethanol, and the mixtures were agitated until clear solutions were obtained. The formulations F(III)-1, F(III)-2 and F(III)-3 obtained have the HLB values of 13.0, 12.4, and 12.0, respectively. 1165 mg, 1300 mg, and 1430 mg respectively of the clear solutions of the three formulations were filled into capsules for further tests.

### Example 5

| Ingredients | mg/capsule | | |
|---|---|---|---|
| | F(IV)-1 | F(IV)-2 | F(IV)-3 |
| Cyclosporine | 100.0 | 100.0 | 100.0 |
| EtOH | 117.5 | 117.5 | 117.5 |
| Labrafil M1944CS | 84.5 | 189.5 | 324.5 |
| Tween80 | 758.0 | 758.0 | 758.0 |
| TOTAL | 1060 | 1165 | 1300 |

Cyclosporine was added into and mixed with Labrafil M1944CS, Tween80 and ethanol, and the mixtures were agitated until clear solutions were obtained. The formulations F(IV)-1, F(IV)-2 and F(IV)-3 obtained have the HLB values of 13.1, 12.2, and 11.3, respectively. 1060 mg, 1165 mg, and 1300 mg respectively of the clear solutions of the three formulations were filled into capsules for further tests.

### Example 6

| Ingredients | mg/capsule | | |
|---|---|---|---|
| | F(V)-1 | F(V)-2 | F(V)-3 |
| Cyclosporine | 100.0 | 100.0 | 100.0 |
| EtOH | 117.5 | 117.5 | 117.5 |
| Labrafil M1944CS | 36.0 | 166.5 | 242.0 |
| Tween80 | 758.0 | 666.0 | 590.0 |
| TOTAL | 1011.5 | 1050 | 1049.5 |

Cyclosporine was added into and mixed with Labrafil M1944CS, Tween80 and ethanol, and the mixtures were agitated until clear solutions were obtained. The formulations F(V)-1, F(V)-2 and F(V)-3 obtained have the HLB values of 13.6, 12.2, and 11.3, respectively. 1011.5 mg, 1050 mg, and 1049.5 mg respectively of the clear solutions of the three formulations were filled into capsules for further tests.

### Example 7

| Ingredients | mg/capsule |
|---|---|
| | Tacrolimus F(I) |
| Tacrolimus | 1.0 |
| EtOH | 8.7 |
| Labrafil M1944CS | 17.3 |
| Tween80 | 43.0 |
| TOTAL | 70 |

Tacrolimus was added into and mixed with Labrafil M1944CS, Tween80 and ethanol, and the mixture was agitated until a clear solution was obtained. The formulation Tacrolimus F(I) obtained has the HLB values of 11.3. 70 mg of the clear solution of the formulation were filled into capsules for further tests.

### Example 8

| Ingredients | mg/capsule |
|---|---|
| | Tacrolimus F(II) |
| Tacrolimus | 5.0 |
| EtOH | 43.5 |
| Tween80 | 301.5 |
| TOTAL | 350 |

Tacrolimus was added into and mixed with Tween80 and ethanol, and the mixture was agitated until a clear solution was obtained. The formulation Tacrolimus F(II) obtained has the HLB values of 14.1. 350 mg of the clear solution of the formulation were filled into capsules for further tests.

### Example 9

| Ingredients | mg/capsule |
|---|---|
| Ketoprofen | 100.0 |
| EtOH | 118.0 |
| Tween80 | 758.0 |
| TOTAL | 976.0 |

Ketoprofen was added into and mixed with Tween80 and ethanol, and the mixture was agitated until a clear solution was obtained. The formulation Ketoprofen obtained has the HLB values of 14. 976 mg of the clear solution of the formulation were filled into capsules for further tests.

### Example 10 Dissolution Test

The dissolution test was conducted by the following procedures:
Instrument: Logan UDT-804 (USA)
Medium: 500 ml of 0.1N HCl solution were prepared by adding 8.5 ml of 37% concentrated HCl into 900 ml of distilled water followed by homogeneously agitating and mixing the solution and bringing it up the total volume to 1 L.
USP Apparatus 2 (Paddle): 50 rpm
Temperature: 37 ± 0.5°C
Sampling time (min): 7, 15, 30, 45, 60, 90, and 120
Procedures:
   i. 500 ml of 0.1N HCl solution were poured into each of the 6 dissolution vessel to be preheated. The temperature of the water bath chamber was maintained at 37 ± 0.5°C and the agitation equipment of the dissolution tester was set up according to one's needs.
   ii. The temperature of the dissolution medium was measured by a thermometer. The dissolution test was conducted once the temperature reached 37 ± 0.5°C.
   iii. One capsule of each formulation was dropped into a dissolution vessel.
   iv. The samples at the following time points: 7 min, 15 min, 30 min, 45 min, 60 min, 90 min, and 120 min were collected.
   v. The samples were filtrated with filters made of PVDF with a pore size of 0.45 µm, and then the drug concentration of the samples was measured by HPLC.

The HPLC measurement was conducted with the following equipment and conditions:
i. Mobile phase: A mixture of distilled water : acetonitrile : methyl tert-butyl ether : phosphoric acid (v/v/v/v) = 440 : 510 : 45 : 1 was prepared. The mixture was filtrated with a filter made of Nylon 66 with a pore size of 0.45 µm. The mixture was sonicated for at least 30 minutes by an ultrasonic sonicator so as to remove the gas contained in the liquid.
ii. Pump (Pump L-7100, HITACHI, JAPAN)- flow rate: 1.5 ml/min
iii. Detector (UV Detector L-7400, HITACHI, JAPAN)- wavelength: 210-nm
iv. The sample vials were positioned on the sample rack of the autosampler (Autosampler L-7200, HITACHI, JAPAN) and the injection volume was set to 20 µl.
v. Column: 4.6-mm x 250 cm column that contains USP packing L1.
vi. Column Temperature: The column was placed in a column oven of 80°C.

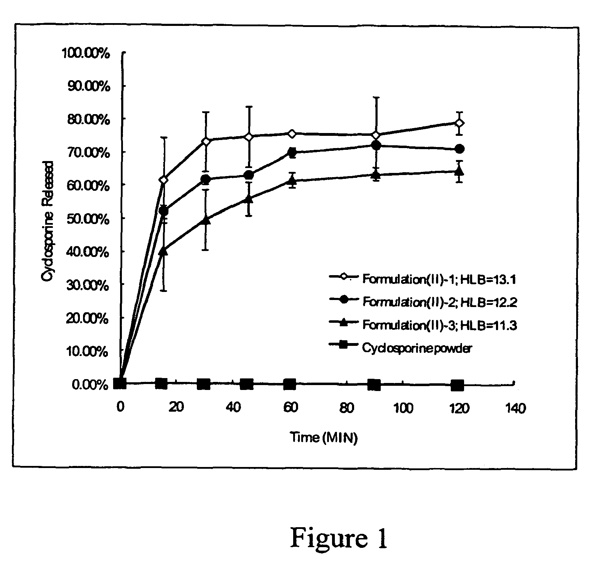

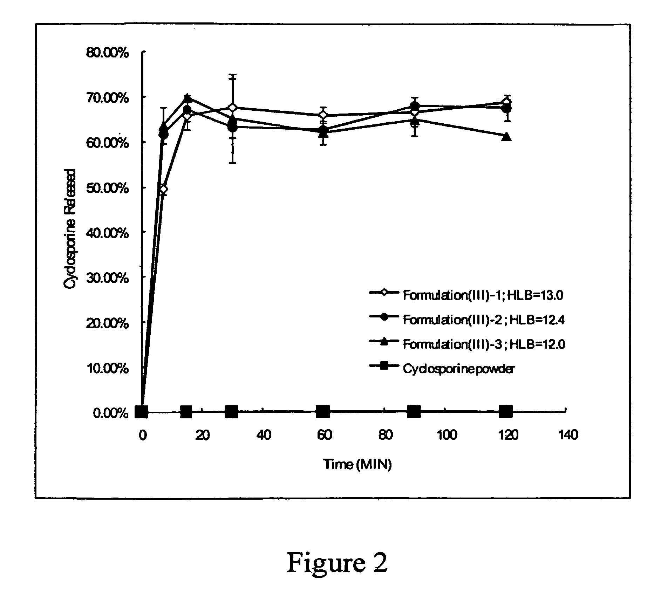

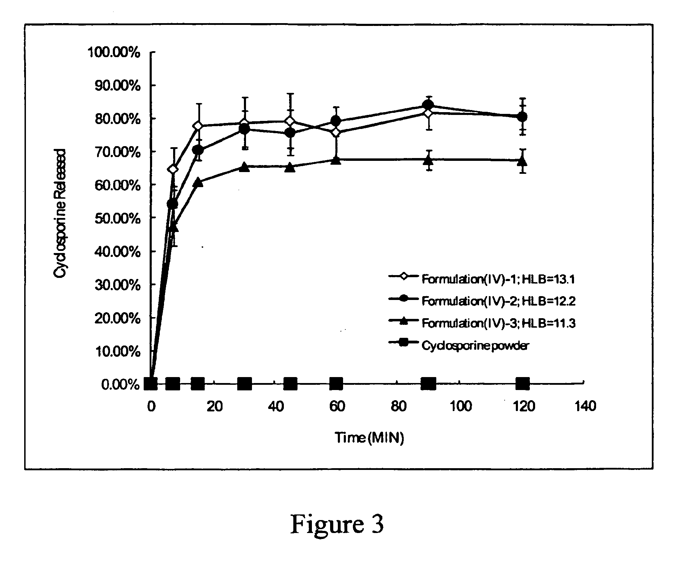

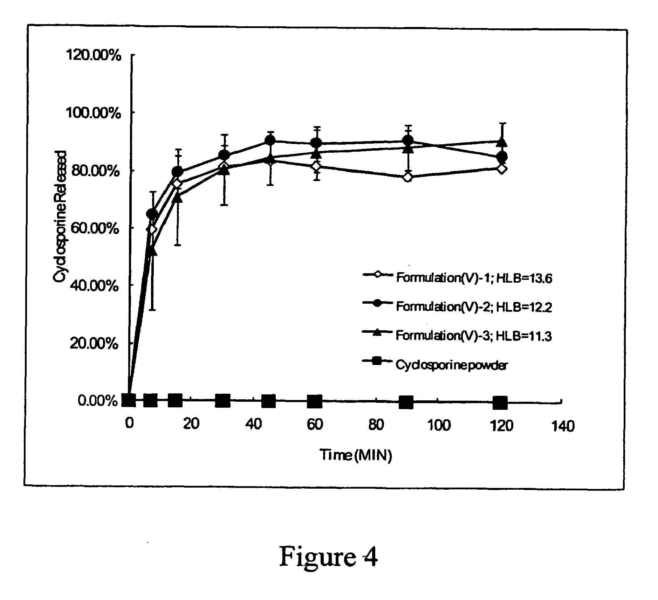

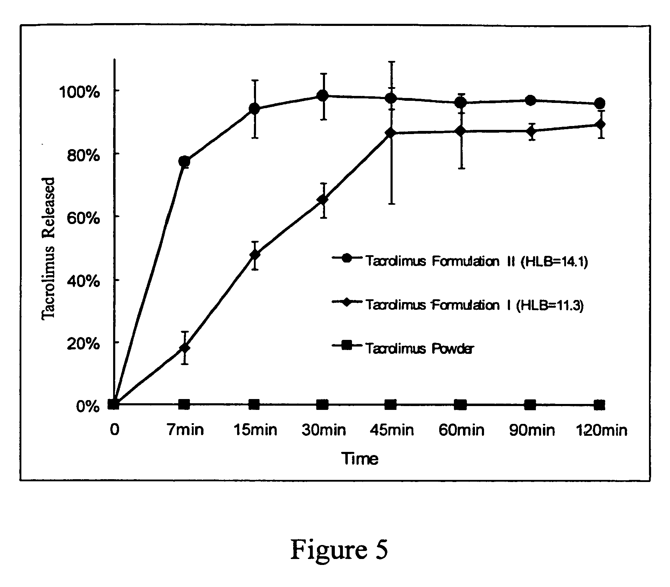

As the results of the dissolution test in Figures 1 to 5 show, the drugs contained in the pharmaceutical products prepared according to the invention can be efficiently dissolved and released therefrom, and the dissolution can be controlled by adjusting the HLB value of the drug delivery system.

### Example 11 Measurement of Particle Sizes of Microemulsions

The particle size of the microemulsions formed by the formulations of Examples 3 to 8 in the 0.1 N HCl solution (an artificial gastric fluid) was measured by the following procedures:
i. 500 ml of 0.1 N HCl (the dissolution medium) were poured into the dissolution vessel and heated to 37°C.
ii. Once the temperature reached 37°C, 1 ml of the solution of each formulation was added into the dissolution vessel.
iii. The mixture was agitated by paddle at 50 rpm for 30 minutes.
iv. About 3 ml of the mixture were taken and added into a sample cuvette, and then the particle sizes of the microemulsions formed were measured in a dynamic light scattering (Zetasizer 3000, Malvern Inst., UK) by following the instructions given in the manuals provided by the manufacturer.

The resulting of particle sizes of the microemulsions formed by the formulations of the present invention are shown in Table 1.

**Table 1**

| Formulations | Particle Sizes (nm) (MEAN±SD) |
|---|---|
| Cyclosporine F(I)-1 | 652.3±11.8 |
| Cyclosporine F(I)-2 | 733.8±28.4 |
| Cyclosporine F(III)-1 | 733.6±34.0 |
| Cyclosporine F(III)-2 | 601.4±13.8 |
| Cyclosporine F(III)-3 | 570.6±13.8 |
| Cyclosporine F(V)-1 | 271.4±18.9 |
| Cyclosporine F(V)-2 | 236.4±4.0 |
| Cyclosporine F(V)-3 | 230.4±12.5 |
| Tacrolimus F(I) | 290.3±9.0 |
| Ketoprofen | 9.5±2.7 |

As shown in Table 1, the cyclosporine formulations of Example 1, which have various ethanol ratios and a single surfactant, all spontaneously formed microemulsions in the artificial gastric fluid. The cyclosporine formulations of Examples 4 and 6, which have various species and ratios of surfactants and HLB values between 8 and 15, are all capable of spontaneously forming microemulsions in the artificial gastric fluid. The formulations of Examples 7 and 9, which have various drugs, all exhibit good self-emulsion capability. The above results suggest that the drug delivery system of the present invention can be applied to various drugs or active ingredients.

### Example 12 Stability Test

The particle size of the microemulsions formed by the formulations of Examples 3 to 8 in the 0.1 N HCl solution (an artificial gastric fluid) was measured by the following procedures:

The stability test was conducted according to the ICH Guidelines. The capsules filled with F(V)-3 were individually placed in different thermohygrostats with three different temperatures and humidities, set to 25°C/60%±5%RH, 30°C/60%±5%RH, and 40°C/75%±5%RH. The remaining contents of the major ingredients were analyzed according to the above HPLC method after 0, 2and 3 months of storage. The results of the test are shown in Table 2.

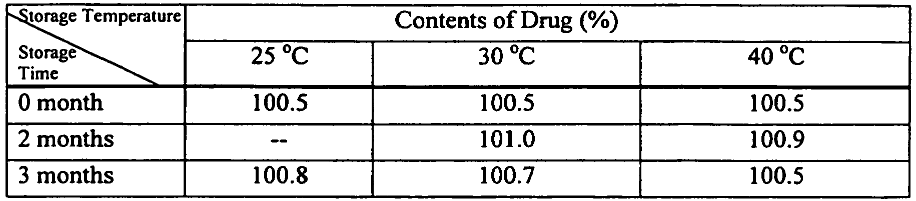

As shown in Table 2, the active ingredient in the pharmaceutical composition of the invention is very stable after long-term storage, even with a temperature of 40 °C.

### Example 13 Oral Bioavailability Test

The bioavailability test was performed in 3 healthy young male volunteers for each of the cyclosporine drugs containing F(I)-1(hard capsule), F(I)-1(soft capsule) and F(V)-3. A fter an overnight fast, a single dose of the test formulations was administered to the volunteers, and the blood samples of the subjects were collected before the dose, (0 h) and at 0.5h, 1h, 1.33h, 1.67h, 2h, 2.5h, 3h, 4h, 6h, 9h, 12h and 24h after drug administration. Plasma concentrations of cyclosporine were determined by high-performance liquid chromatography with mass detection (LC-Mass).

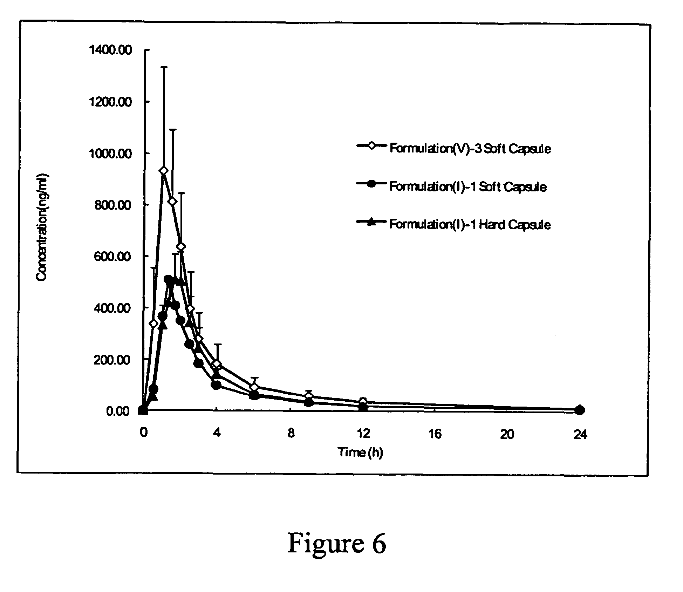

Figure 6 reveals that all the cyclosporine Formulations (I)-1 and (V)-3 have a good oral bioavailability of cyclosporine in humans.

### REFERENCES

US 6,436,430 B1
US 6,312,704 B1
US 6,057,289
US 5,965,160
US 5,993,858
US 6,054,136
US 6,458,373 B1
US 6,596,306 B1
US 6,638,522 B1
US 7,094,804 B2
US 6,960,563 B2

## Claims

1. An oral self-emulsifying pharmaceutical composition, which comprises
(a) a therapeutically effective amount of a lipophilic drug;
(b) polysorbate;
(c) oleoyl polyoxyglycerides; and
(d) one or more hydrophilic carriers selected from the group consisting of ethanol, isopropanol, polyethylene glycol (PEG), glycerine, propylene glycol, and combinations thereof, wherein the hydrophilic lipophilic balance value (HLB value) of said composition ranges from about 8 to about 15, and the composition does not contain an oil phase.

2. The oral self-emulsifying pharmaceutical composition of Claim 1, wherein the drug is an immune agent, an anti-infection agent, an anti-hypertensive agent, or a blood lipid-lowering agent.

3. The oral self-emulsifying pharmaceutical composition of any one of Claim 1 or 2, wherein the drug is cyclosporine, tacrolimus, ibuprofen, ketoprofen, nifedipine, amlodipine, or simvastatin.

4. The oral self-emulsifying pharmaceutical composition of any one of the preceding claims, wherein said composition forms emulsions/microemulsions with a particle size of less than about 800 nm when said composition is contacted with an aqueous solution.

5. The oral self-emulsifying pharmaceutical composition of any one of the preceding claims, wherein the drug is present in an amount of about 0.1% to about 50% by weight of the composition.

6. The oral self-emulsifying pharmaceutical composition of any one of the preceding claims, wherein the hydrophilic carrier is present in an amount of about 1% to about 30% by weight of the composition.

7. The oral self-emulsifying pharmaceutical composition of any one of the preceding claims, wherein the surfactant is present in an amount of about 10% to about 90% by weight of the composition.

8. A method of preparing the oral self-emulsifying pharmaceutical composition according to any one of Claims 1 to 7, comprising mixing the lipophilic drug, polysorbate, oleoyl polyoxyglycerides and the hydrophilic carrier by agitation.

9. The method according to Claim 8, which comprises the steps of dissolving the drug in a mixture comprising polysorbate, oleoyl polyoxyglycerides and the hydrophilic carrier, thereby the composition having the HLB value ranging from about 8 to about 15 is obtained.

10. The method according to Claim 8 or 9, which further comprises the step of encapsulating the composition in a sealed soft or hard capsule.

11. The oral self micro-emulsifying pharmaceutical composition of any of claims 1-7, wherein the lipophilic drug is cyclosporine.

12. The oral self micro-emulsifying pharmaceutical composition of any of claims 1-7 and 11, wherein the hydrophilic carrier(s) is ethanol.

## Patentansprüche

1. Orale selbst-emulgierende pharmazeutische Zusammensetzung, umfassend
(a) eine therapeutisch wirksame Menge eines lipophilen Arzneimittels;
(b) Polysorbat;
(c) Oleoylpolyoxyglyceride; und
(d) einen oder mehrere hydrophile Träger ausgewählt unter der Gruppe bestehend aus Ethanol, Isopropanol, Polyethylenglycol (PEG), Glycerin, Propylenglycol, und Kombinationen davon, worin der hydrophilic-lipophilic-balance Wert (HLB Wert) der Zusammensetzung von ungefähr 8 bis ungefähr 15 reicht, und die Zusammensetzung keine Ölphase umfasst.

2. Orale selbst-emulgierende pharmazeutische Zusammensetzung nach Anspruch 1, worin das Arzneimittel ein Immunagens, ein Antiinfektionsagens, ein antihypertensives Agens, oder ein Agens zur Blutlipidsenkung ist.

3. Orale selbst-emulgierende pharmazeutische Zusammensetzung nach einem der Ansprüche 1 oder 2, worin das Arzneimittel Cyclosporin, Tacrolimus, Ibuprofen, Ketoprofen, Nifedipin, Amlodipin, oder Simvastatin ist.

4. Orale selbst-emulgierende pharmazeutische Zusammensetzung nach einem der vorstehenden Ansprüche, worin die Zusammensetzung Emulsionen/Mikroemulsionen mit einer Teilchengröße von weniger als ungefähr 800 nm bildet, wenn die Zusammensetzung mit einer wässrigen Lösung in Kontakt gebracht wird.

5. Orale selbst-emulgierende pharmazeutische Zusammensetzung nach einem der vorstehenden Ansprüche, worin das Arzneimittel in einer Menge von ungefähr 0,1 bis ungefähr 50 Gew.-% der Zusammensetzung vorliegt.

6. Orale selbst-emulgierende pharmazeutische Zusammensetzung nach einem der vorstehenden Ansprüche, worin der hydrophile Träger in einer Menge von ungefähr 1 bis ungefähr 30 Gew.-% der Zusammensetzung vorliegt.

7. Orale selbst-emulgierende pharmazeutische Zusammensetzung nach einem der vorstehenden Ansprüche, worin das grenzflächenaktive Mittel in einer Menge von ungefähr 10 bis ungefähr 90 Gew.-% der Zusammensetzung vorliegt.

8. Verfahren zur Herstellung einer oralen selbst-emulgierenden pharmazeutischen Zusammensetzung nach einem der Ansprüche 1 bis 7, umfassend Mischen des lipophilen Arzneimittels, Polysorbats, der Oleoylpolyoxyglyceride und des hydrophilen Trägers durch Rühren.

9. Verfahren nach Anspruch 8, das die Schritte eines Lösens des Arzneimittels in einem Gemisch umfasst, das Polysorbat, Oleoylpolyoxyglyceride und den hydrophilen Träger umfasst, wodurch die Zusammensetzung mit dem HLB Wert in dem Bereich von ungefähr 8 bis ungefähr 15 erhalten wird.

10. Verfahren nach Anspruch 8 oder 9, das ferner den Schritt eines Einkapseln der Zusammensetzung in einer dichten Weich- oder Hartkapsel umfasst.

11. Orale selbst-mikroemulgierende pharmazeutische Zusammensetzung nach einem der Ansprüche 1-7, worin das lipophile Arzneimittel Cyclosporin ist.

12. Orale selbst-mikroemulgierende pharmazeutische Zusammensetzung nach einem der Ansprüche 1-7 und 11, worin der/die hydrophile(n) Träger Ethanol ist.

## Revendications

1. Une composition pharmaceutique orale autoémulsifiante qui comprend :
(a) une quantité thérapeutiquement active d'un médicament lipophile ;
(b) du polysorbate ;
(c) des oléoyl polyoxyglycérides ; et
(d) un ou plusieurs supports hydrophiles choisis à partir du groupe consistant en éthanol, isopropanol, polyéthylène glycol (PEG), glycérine, propylène glycol et des combinaisons de ceux-ci, dans laquelle la balance hydrophile-lipophile (valeur HLB) de ladite composition se situe entre environ 8 et environ 15 et la composition ne contient pas une phase huile.

2. La composition pharmaceutique orale autoémulsifiante de la revendication 1 dans laquelle le médicament est un agent immune, un agent anti-infectieux, un agent anti-hypertenseur ou un agent hypolipidémiant.

3. La composition pharmaceutique orale autoémulsifiante de l'une des revendications 1 ou 2 dans laquelle le médicament est cyclosporine, tacrolimus, ibuprofène, kétoprofène, nifédipine, amlodipine ou simvastatine.

4. La composition pharmaceutique orale autoémulsifiante de l'une des revendications précédentes dans laquelle ladite composition forme des émulsions / microémulsions avec une taille de particules de moins environ 800 nm lorsque ladite composition est mise en contact avec une solution aqueuse.

5. La composition pharmaceutique orale autoémulsifiante de l'une des revendications précédentes dans laquelle le médicament est présent en une quantité d'environ 0.1 % à environ 50 % du poids de la composition.

6. La composition pharmaceutique orale autoémulsifiante de de l'une des revendications précédentes dans laquelle le support hydrophile est présent eu une quantité d'environ 1 % é environ 30 % du poids de la composition.

7. La composition pharmaceutique orale autoémulsifiante de de l'une des revendications précédentes dans laquelle le surfactant est présent en une quantité d'environ 10 % à environ 90 % du poids de la composition.

8. Un procédé de préparation de la composition pharmaceutique orale autoémulsifiante selon l'une des revendications 1 à 7 comprenant le mélange par agitation du médicament lipophile, du polysorbate, des oléoyl polyoxyglycérides et du support hydrophile.

9. Le procédé selon la revendication 8 qui comprend les étapes de dissoudre le médicament dans un et que l'on obtient ainsi la composition ayant une valeur HLB allant d'environ 8 à environ 15.

10. Le procédé selon la revendication 8 ou 9 qui comprend en outre l'étape d'encapsulation de la composition dans une capsule scellée molle ou dure.

11. La composition pharmaceutique autoémulsifiante de l'une des revendications 1 à 7 dans laquelle le médicament lipophile est la cyclosporine.

12. La composition autoémulsifiante de l'une des revendications 1 à 7 et 11 dans laquelle le support hydrophile est l'éthanol.
